# FASCICULE DE BREVET EUROPEEN

(11) **EP 1 578 709 B1**
(45) Date de publication et mention de la délivrance du brevet: **16.10.2019**
(21) Numéro de dépôt: 03815415.9
(22) Date de dépôt: 17.12.2003
(51) Int. Cl.: C07C 41/09, C07C 43/13, C08G 65/34, C07H 3/00, H05B 6/80, H05B 6/46

(54) **PROCÉDÉ DE POLYCONDENSATION PAR CHAUFFAGE DIÉLECTRIQUE, NOTAMMENT POUR LA PRODUCTION DE POLYGLYCÉROLS ET ANALOGUES**
VERFAHREN ZUR POLYKONDENSATION UNTER DIELEKTRISCHER ERWÄRMUNG, INSBESONDERE ZUR HERSTELLUNG VON POLYGLYCEROLEN UND ANALOGEN
POLYCONDENSATION METHOD EMPLOYING DIELECTRIC HEATING, IN PARTICULAR FOR THE PRODUCTION OF POLYGLYCEROLS AND SIMILAR

(30) Priorité: 23.12.2002 FR 0216741
(43) Date de publication de la demande: 28.09.2005
(73) Titulaire: Aldivia S.A., 69230 Saint Genis-Laval (FR)
(72) Inventeur: CHARLIER DE CHILY, Pierre, F-60540 Irigny (FR); RAYNARD, Mikaele, 44730 Saint-Michel Chef Chef (FR)
(74) Mandataire: Lavoix
(86) Numéro de dépôt international: PCT/FR2003/003755
(87) Numéro de publication internationale: WO 2004/065343

(56) Documents cités:
- EP-A- 0 518 765
- WO-A-95/16723
- GB-A- 494 639

## Description

### Secteur technique de l'invention :

Les polymères d'alcools polyhydriques trouvent de multiples applications.

Les polymères comportant des fonctions hydroxyles sont utilisés, par exemple, comme humectants en cosmétique ou comme intermédiaires de synthèse (alcools) dans les réactions d'estérification ou de transestérification.

Ils permettent en effet la fabrication d'esters de polymères d'alcools polyhydriques qui constituent des tensio-actifs non ioniques, non agressifs de plus en plus demandés sur le marché. Ces derniers permettent de remplacer par exemple les tensio-actifs polyéthoxylés, irritants pour la peau : ces tensio-actifs contiennent des dérivés d'éthylène : les dioxanes, connus pour leur effet irritant.

### Problème posé :

Les procédés d'obtention des polymères d'alcools polyhydriques et/ou d'alcools monohydriques sont connus de l'homme de l'art. Néanmoins, ces procédés sont longs, coûteux et nécessitent l'emploi de catalyseurs, souvent toxiques.

La présente invention consiste en la fabrication des polymères d'alcools polyhydriques selon un procédé non toxique avec des gains de temps considérables.

La fabrication des esters de polymères d'alcools polyhydriques fait l'objet d'une demande de brevet FR 20020005396 (publié sous le numéro FR2839069).

### Art antérieur :

### 1- Intérêt des esters de polymères d'alcools polyhydriques.

Les polymères comportant des fonctions hydroxyles permettent la fabrication d'esters de polymères d'alcools polyhydriques qui constituent des tensio-actifs non ioniques, non agressifs de plus en plus demandés sur le marché.

Les tensio-actifs sont des molécules possédant au moins un groupement ayant une affinité pour l'eau (partie hydrophile) et un radical ayant une affinité pour l'huile (partie lipophile). Ils s'adsorbent aux interfaces et font chuter la tension superficielle.

Les tensio-actifs ont des propriétés détergentes, mouillantes, solubilisantes, émulsionnantes.

Les tensio-actifs sont donc des agents de surface qui vont permettre à deux phases non solubles l'une dans l'autre de former un système stable. Les agents de surface vont former un film à la surface du liquide pour réduire la tension de surface. Lorsque la concentration en agent de surface est telle que l'interface est saturée, les parties hydrophiles vont se tourner vers l'extérieur et les parties lipophiles vers l'intérieur pour former une micelle.

Il existe deux catégories de tensio-actifs :
- Les tensio-actifs ioniques (anionique, cationique et amphotère) qui possèdent une charge à leur surface, et qui sont donc agressifs et irritants pour la peau et les muqueuses.
- Les tensio-actifs non ioniques composés d'une chaîne grasse plus ou moins longue ; ce sont surtout des émulsionnants, des mouillants, des solubilisants. On retrouve notamment les esters de sorbitan et les alcools gras polyoxyéthylénés. Ces produits peuvent contenir des dérivés d'oxyde d'éthylène : les dioxanes, irritants pour la peau. Ces derniers peuvent également pénétrer la barrière cutanée et provoquer des cancers du foie et des voies nasales.

### 2- Procédés de fabrication des polymères d'alcools polyhydriques.

### a- fabrication des polyglycérols

Les procédés de production de polyglycérols sont nombreux.

Dans le brevet anglais GB 494,639 déposé le 20 janvier 1938 par Herbert Schou, le glycérol exempt d'eau est chauffé en présence d'un gaz inerte (dioxyde de carbone, azote,...), et d'un catalyseur (comme l'acétate de soude, 2%), pendant 72 heures. Le polyglycérol obtenu a une viscosité à 50°C supérieure ou égale à 1000 centipoises.

Les temps de réactions sont très longs (72h)
Le brevet 2,455,327 consiste à produire des polyglycérols par condensation du glycérol sous 20 mm Hg en présence d'acide sulfurique et d'acétate de glycérol. Les temps de réaction sont plus courts mais la formation de sous-produits tels que l'acroléine et les esters d'acide sulfurique rend ce procédé désavantageux.

Le brevet DE 1987 00 37 21 003 du 25 juin 1987 de Deutsche Solvay-Werke GmbH consiste à produire des polyglycérols en 4 étapes en utilisant du chlorure d'hydrogène gazeux ou de l'acide chlorhydrique concentré à des températures comprises entre 340°K et 410°K. Ce procédé évite la formation de sous-produits cycliques. Néanmoins, il reste complexe et utilise des catalyseurs toxiques.

Le brevet WO 95/16723 du 22 juin 1995 de Unichema Chemie consiste à produire des polyglycérols à partir du glycérol chauffé à 240°C en présence d'hydrotalcite, et/ou de pyroaurite, et/ou de tacovite, et/ou de stichtite, et/ou de reevesite, et/ou eardlegite, et/ou de meixnerite. Il faut 11 heures pour obtenir un polyglycérol avec un degré de polymérisation égal à 3 (majorité de glycérol) et 30 à 35 heures pour obtenir un polyglycérol avec un degré de polymérisation égal à 5 (majorité de diglycérol). Ce procédé permet d'obtenir une majorité de polyglycérols linéaires mais le temps de réaction est très long.

Le brevet EP0719752A1 du 27 décembre 1995 de l'Institut Français du Pétrole consiste à produire des polyglycérols par condensation du glycérol contenant au moins un sel minéral (sulfate, chlorure ou phosphate de métal alcalin), en présence d'au moins un catalyseur basique. Ce procédé comprend de nombreuses étapes : neutralisation des ions alcalins provenant du catalyseur basique et des sels présents dans la matière première glycérineuse par addition d'acide sulfurique, précipitation du sulfate insoluble par ajout d'alcool, filtration... Ces étapes sont longues et coûteuses.

Le brevet n°74 39087 du 28 novembre 1974 de The Procter & Gamble Company consiste à produire des polyglycérols à partir de glycérol sous une pression absolue de 400mm de mercure à 110-180°C en présence d'acide sulfurique et d'un acide aliphatique inférieur. Ce procédé utilise des catalyseurs toxiques.

Le brevet EP0151755A1 du 12 décembre 1984 de Hoechst consiste à produire des polyglycérols entre 190 et 250°C en présence de catalyseurs phosphoriques (acide hypophosphorique H3PO2, acide phosphorique H3PO3) et de métaux alcalins (soude NaOH, potasse KOH, acétate de sodium CH3ONa, carbonate de sodium Na2CO3...). Dans ce cas, les temps réactionnels sont très longs (exemple : 72h à 250°C pour obtenir un polyglycérol avec un indice d'hydroxyle égale à 1360. Cette valeur correspond à celle du diglycérol)

### b- fabrication des polyglucosides et alkyl polyglucosides

Les procédés de production d'alkyl polyglucosides sont connus de l'homme de l'art :
Le brevet EP 0570056 A du 6 mai 1993 de ENICHEM décrit un procédé de fabrication d'alkyl polyglucosides. Cette synthèse est réalisée en présence d'un catalyseur binaire constituée d'un acide fort (comme les acides alkylbenzenesulfoniques, les acides alkylsulfoniques secondaires et tertiaires) et d'une base faible (comme la pyridine, la quinoline, la picoline) à 90-130°C pendant des temps allant de 4 à 6 heures. Des étapes supplémentaires ont lieu en fin de synthèse : ajout d'une base forte, distillation...

Le brevet EP 0913403A1 du 22 octobre 1998 de CONDEA AUGUSTA consiste en la fabrication d'alkyl polyglucosides en présence de catalyseurs acides, les acides polyalkylarylsulfoniques, à 110-130°C sous 25mmHg pendant 6 heures.

Le brevet DE 10 13 902 du 24 mai 1961 de FARBWERKE HOECHST AktienGesellschaft consiste à produire des polysaccharides en présence d'alkyl esters d'acides polyphosphoriques, dans un solvant tels que le diméthylformamide, le diméthylsufoxyde à 60°C pendant 6 heures. A la fin de la synthèse, des étapes supplémentaires sont nécessaires : précipitation, dissolution dans l'eau. Ces étapes sont longues : elles durent plus de 48 heures.

Ces procédés comprennent de nombreuses étapes et utilisent des catalyseurs souvent toxiques.

### Résumé de l'invention :

La présente invention propose un nouveau procédé industriel, permettant la polycondensation d'alcools polyhydriques d'origine naturelle ou synthétiques, seuls ou en mélange. Ce procédé utilise les ondes électromagnétiques. Il est non toxique et permet des gains de temps considérables.

### Description de l'invention

La présente invention propose un procédé industriel nouveau, utilisant les ondes électromagnétiques pour fabriquer des polymères d'alcools notamment les polyglycérols.

Ce procédé permet de réduire de manière tout à fait considérable les temps de réaction et d'économiser de l'énergie avec des coûts d'investissement plus faibles. Les bénéfices de la présente invention sont :
1- de réduire de manière significative les temps de réaction ;
2- de réaliser la réaction en une seule étape ;
3- de ne pas utiliser de solvant
4- d'économiser de l'énergie (car les temps sont significativement plus courts) ;
5- d'éviter le « burn-up » et les sous-réactions indésirées.

Le Demandeur a déposé une demande de brevet FR 98 13770 et une demande de brevet PCT WO 00/26265 (PCT/FR99/02646) concernant un procédé original de chauffage diélectrique. Ce procédé s'applique particulièrement à la préparation de polymères d'alcools polyhydriques.

### 1 - Principe du chauffage diélectrique

On utilisera le chauffage diélectrique, c'est à dire un chauffage sous fréquences micro-ondes ou hautes fréquences, comme décrit dans les brevet précités, dont on donne ci-dessous un extrait pour la convenance de l'homme de métier, et auquel celui-ci pourra se reporter utilement pour les détails de mise en oeuvre.

Selon ces brevets, le procédé de polymérisation d'alcools polyhydriques est caractérisé par le fait que le réactif ou le mélange réactionnel est soumis à un chauffage diélectrique pour opérer la polymérisation.

### De préférence :

Le chauffage est effectué par utilisation de fréquences micro-ondes ou bien par utilisation de fréquence radio.

Il est mis en oeuvre avec ou sans catalyseur.

On peut ajouter au réactif ou au mélange réactionnel des catalyseurs hétérogènes ou homogènes.

On peut ajouter au réactif ou au mélange réactionnel des catalyseurs répondant aux fréquences radio ou aux fréquences micro-ondes, tel que l'acétate de sodium.

Le réactif ou mélange réactionnel, et éventuellement le(s) catalyseur(s), est placé dans un réacteur de type batch ou discontinu adapté pour recevoir des fréquences micro-ondes ou fréquences radio.

Le réactif ou le mélange réactionnel, et éventuellement le(s) catalyseur(s), peut aussi être placé dans un réacteur adapté pour faire des réactions en continu.

Les fréquences sont comprises entre environ 30 GHz et environ 300 MHz.

Les fréquences sont 2.45 GHz ou 915 MHz

Les fréquences sont comprises entre environ 300 MHz et environ 3 MHz.

Les fréquences sont 13.56 MHz ou 27.12 Mhz

### 2- Le procédé de polycondensation

L'invention consiste à soumettre des alcools polyhydriques à un champ électromagnétique et à les condenser pour former des polymères. On soumet les réactifs, seuls ou en mélange, à des ondes électromagnétiques choisies dans les fréquences allant de environ 30 GHz à environ 3 MHz.

Les fréquences micro-ondes sont comprises entre environ 300 Mhz et environ 30 Ghz, préférentiellement à 915 MHz (fréquence autorisée avec une tolérance de 1.4%) ou à 2.45 GHz (fréquence autorisée avec une tolérance de 2%)

Les hautes fréquences sont comprises entre environ 3 MHz et environ 300 MHz, préférentiellement à 13.56 MHz (fréquence autorisée avec une tolérance de 0.05%) ou à 27.112 MHz (fréquence autorisée avec une tolérance de 0.6%).

Les températures de réaction se situent entre 60 et 300 °C et mieux encore entre 160 et 260 °C, sous agitation permanente et préférentiellement sans catalyseur. On réalise l'opération sous atmosphère normale ou riche en oxygène ou de préférence en présence de gaz inerte (dioxide de carbone, azote, argon,...) sous pression atmosphérique ou sous pression réduite de préférence entre 10 et 50 mm de mercure, si l'on désire extraire complètement les molécules d'eau.

Le temps de montée en température est choisi entre 3 et 180 minutes.

Le temps total de réaction est fonction du dispositif utilisé, de la température atteinte et du produit que l'on souhaite obtenir (autrement dit du degré de polymérisation que l'on souhaite (atteindre) est compris entre 1 heure et 10 heures.

Les polymères d'alcools polyhydriques et/ou d'alcools monohydriques sont réalisés en une seule étape d'une manière avantageuse ou en plusieurs étapes. On réalise l'opération en batch ou, de manière avantageuse, en continu.

On arrête la polymérisation en laissant refroidir ou en refroidissant le réactif ou le mélange réactionnel à une température inférieure à la température de polymérisation et ce, suivant le polymère que l'on souhaite obtenir.

Le degré de polymérisation DP et la composition du polymère obtenu sont déterminés par diverses analyses : relation entre la quantité d'eau formée au cours de la synthèse et DP, indice de réfraction, viscosité, chromatographie, indice d'hydroxyle...

### 3- Les réactifs

Par la présente invention, les réactifs peuvent être choisis parmi tous les alcools polyhydriques d'origine végétale ou synthétique.

En tant qu'alcool polyhydrique, on peut mentionner, entre autre, le glycérol, le sorbitol, le sucrose, le mannitol, le xylitol, le néopentylglycol, le pentaérythritol, le saccharose, la galactose, le glucose, le maltose, le maltotriose, le fructose, le maltitol, le lactitol, le lactose, le ribose, le mellibiose, le cellobiose, le gentiobiose, l'altrose, le gulose, les polyalkylèneglycols, les polyglycérols, les polyphénols, les alkylpolyglucosides, les polyglucosides, le glycol, le pentaérythritol, l'éthane 1,2 diol, le 1,4 butanediol, 1,6 hexanediol, les aminoalcools (par exemple la diéthanolamine DEA la triéthanolamine TEA, 3-amino 1,2 propanediol), les époxyalcools et leurs analogues....

Les alcools ainsi que leurs dérivés peuvent subir un traitement préalable visant à les rendre plus réactifs ou au contraire moins réactifs comme par exemple l'hydrogénation, l'hydroxylation, l'époxydation, la phosphitation, la sulfonation.

Parmi les catalyseurs ou adjuvants, on entendra à titre d'exemples non limitatifs les catalyseurs acides usuels (acide paratoluènesulfonique, acide sulfurique, acide phosphorique, acide perchlorique...), les catalyseurs basiques usuels (soude, potasse, alcoolate de métaux alcalins et de métaux alcalino-terreux, acétate de sodium, triéthylamines, dérivés de pyridine...), les résines acides et/ou basiques de type Amberlite™, Amberlyst™, Purolite™, Dowex™, Lewatit™, les zéolithes, les enzymes, les noirs de carbone et les fibres de carbone activées.

### Applications :

Le demandeur a découvert un procédé de polycondensation d'alcools polyhydriques pour le marché cosmétique, dermatologique, pharmaceutique, alimentaire et pour l'industrie.

Les polymères d'alcools polyhydriques sont utilisés notamment :
- comme base pour la synthèse d'esters d'alcools polyhydriques
- comme humectant dans la fabrication de produits cosmétiques, dermatologiques, vétérinaires, pharmaceutiques,
- comme fluides hydrauliques ou lubrifiants en industrie.

Parmi ces applications, les polymères comportant des fonctions hydroxyles servent essentiellement de base pour la synthèse d'esters.

Ils peuvent être partiellement transestérifiés avec des huiles végétales ou avec des huiles végétales polymérisées ou oxypolymérisées ou ils peuvent être partiellement estérifiés avec des acides gras saturés ou insaturés pour être utilisés :
- dans des applications cosmétiques comme émollients et hydratants dans la fabrication de bains moussants, shampooings, démaquillants, crèmes de soins pour tout type de peaux, crèmes pour le corps, lotions...
- dans des applications pharmaceutiques et dermatologiques dans la fabrication d'onguents, de produits dermatologiques...
- dans des applications alimentaires comme additifs alimentaires, comme produits de base d'émulsifiants (chocolats, margarine..), comme alicaments...
- dans des applications industrielles comme agents épaississants, agents anti-usure dans la fabrication de lubrifiants solubles dans l'eau

Cette liste n'est pas exhaustive. Ces polymères d'alcools polyhydriques sont utilisables dans toutes les applications actuelles et futures de ces produits.

Des exemples concrets de l'invention vont être maintenant présentés.

### EXEMPLES

Les exemples suivants sont réalisés à une fréquence de 2,45 Ghz ou de 915 MHz.

Comme indiqué dans ce présent brevet, le chauffage diélectrique, c'est à dire le chauffage sous fréquences micro-ondes ou hautes fréquences, permet des gains de temps et d'énergie, combinés à un coût d'investissement plus faible.

Les tableaux ci-dessous montrent l'efficacité du chauffage diélectrique comparé au chauffage classique.

### 1- COMPARAISON AVEC SCHOU (GB 494,639)

Les exemples suivants sont réalisés en présence d'acétate de sodium, selon le procédé de SCHOU. Seul le mode de chauffage change : micro-onde et classique.

**Tableau n°1 : comparaison chauffage MO et chauffage classique**

| | **Quantité des matières premières** | | **Température (°C)** | **Temps** | | **Viscosité du polyglycérol à 50°C (cP)** |
|---|---|---|---|---|---|---|
| | **glycérol** | **Acétate de sodium** | | **MO (invention)** | **CL (Schou*)** | |
| Exemple 1 | 2 kg | 2% | 260 | 3h | ≥72h | 3600 |
| Exemple 2 | 200 kg | 2% | 260 | 6h30 | | |
| Exemple 3 | 2kg | 2% | 250 | 7h30 | | 4871 |
| Exemple 4 | 2kq | 2% | 270 | 6h00 | | >200 000 |

| | | | | | | |
|---|---|---|---|---|---|---|
| MO : chauffage diélectrique sous une fréquence de 2.45Ghz ou de 915MHz. CL : chauffage classique. * Dans le brevet anglais 494,639 déposé le 20 janvier 1938 par Herbert Schou, le glycérol exempt d'eau est chauffé en présence d'un gaz inerte (dioxyde de carbone, azote,...), et d'acétate de soude (2%), pendant 72 heures. Le polyglycérol obtenu a une viscosité à 50°C supérieure ou égale à 1000 centipoises. | | | | | | |

### 2- COMPARAISON AVEC ONIDOL™

L'exemple suivant est réalisé à 255°C, en présence d'hydroxyde de sodium, selon le procédé de ONIDOL™. Seul le mode de chauffage change : micro-onde et classique.

### a- polyglycérol de ONIDOL™

**Tableau n°2 : nature des polyglycérols obtenus par ONIDOL™**

| **NaOH %/glycérol** | **Température °C** | **temps** | **Produit obtenu par ONIDOL™** | | | | |
|---|---|---|---|---|---|---|---|
| | | | **glycérol** | **diglycérol** | **triglycérol** | **tétraglycérol** | **pentaglycér** |
| 1% | 255 | 6h50 | 22.53 % | 40.9% | 18.63% | %9.4 | 3.22% |

Le produit de ONIDOL contient majoritairement du glycérol, du diglycérol et du triglycérol avec un degré de polymérisation égal à 5.

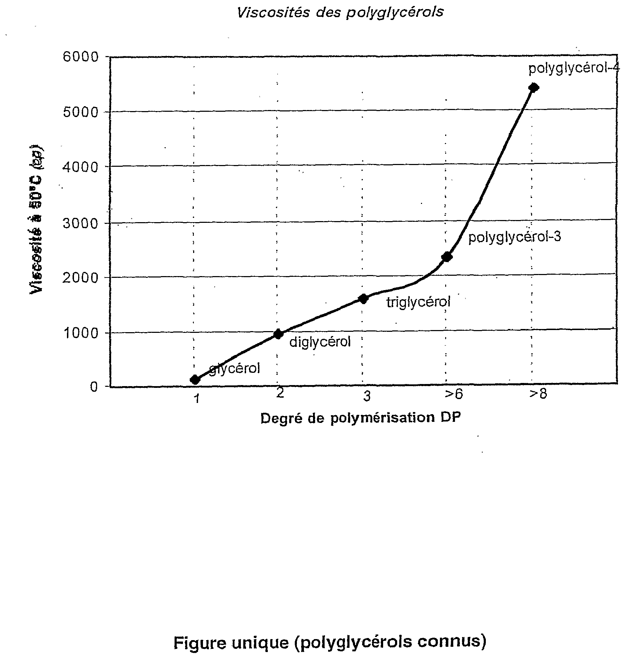

Le graphique représenté en figure unique annexée montre la viscosité de différents polyglycérols connus à 50°C. De ce graphique, on en déduit la viscosité du polyglycérol de ONIDOL™

### Remarques :

Glycérol, diglycérol, triglycérol = Produits purs
Polyglycérol 3 = majorité de triglycérol (45%) et DP >6
Polyglycérol 4 = majorité de tétraglycérol (40%) et DP>8

### b- comparaison entre le chauffage classique et micro-onde

**Tableau n°3 : comparaison chauffage MO et chauffage classique**

| **Chauffage** | **%AcNa/glycérol** | **Température °C** | **Temps** | **Viscosité à 50°C (cP) du polyglycérol** | **Degré de polymérisation** |
|---|---|---|---|---|---|
| Classique ONIDOL™ | 1 | 255 | 6h50 | 2346 | 5 |
| Micro-onde ALDIVIA | 1 | 255 | 6h50 | 33 217 | >8 |

### 3- OBTENTION DE POLYGLYCEROLS DE DEGRE DE POLYMERISATION DIFFERENT

Le temps total de réaction est fonction du dispositif utilisé, de la température atteinte et du produit que l'on souhaite obtenir (autrement dit du degré de polymérisation que l'on souhaite atteindre).

Les exemples suivants sont réalisés à une fréquence de 2,45 Ghz ou de 915 MHz, en présence d'acétate de sodium à 260°C.

**Tableau n°4 : temps réactionnels pour l'obtention de différents polyglycérols**

| **Polyglycérol majoritaire** | **Degré de polymérisation** | **Temps réactionnel** |
|---|---|---|
| diglycérol | 2 | 1h45 |
| triglycérol | 3 | 3h30 |
| triglycérol | 4 | 4h30 |
| triglycérol | 6 | 6h30 |

Remarque : Les temps indiqués sont valables aussi bien pour le traitement thermique de 200 kg de mélange réactionnel.

Comme le comprendra l'homme de métier en fonction des très nombreuses combinaisons de produit / poids moléculaire que l'on peut envisager, on peut indiquer que le temps réactionnel est compris entre environ 1 et 10 h. Pour obtenir un polyglycérol avec un degré de polymérisation = 10, il faudra probablement plus de 10h.

Egalement à titre indicatif, on peut estimer que pour un produit final donné, de poids moléculaire donné, et toutes choses étant égales par ailleurs, le temps de réaction selon l'invention représente entre 5 et 40 %, généralement autour de 10 - 20 % du temps de réaction usuel.

## Revendications

1. Procédé de polycondensation **caractérisé en ce qu'**il comporte le chauffage diélectrique d'alcools polyhydriques pour obtenir des polymères d'alcools polyhydriques ; et est **caractérisé en ce que** :
- le temps de réaction, qui est fonction du dispositif utilisé, de la température atteinte et du produit que l'on souhaite obtenir (autrement dit du degré de polymérisation que l'on souhaite atteindre) est compris entre 1 heure et 10 heures
- les fréquences des ondes électromagnétiques utilisées dans le chauffage diélectrique varient de 30 Ghz à 3 Mhz ;
- la température à laquelle est soumis le réactif ou le mélange réactionnel, et éventuellement le(s) catalyseur(s), est comprise entre 60 et 300°C, préférentiellement entre 160 et 260 °C ; et
- le temps de montée en température est choisi entre 3 et 180 minutes.

2. Procédé selon la revendication 1 **caractérisé en ce qu'**il n'utilise pas de solvant.

3. Procédé selon la revendication 1 **caractérisé en ce que** le chauffage diélectrique est réalisé de préférence dans une atmosphère dépourvue d'oxygène.

4. Procédé selon la revendication 1 **caractérisé en ce que** le chauffage est effectué par utilisation de fréquences choisies parmi les fréquences microondes ou hautes-fréquences.

5. Procédé selon la revendication 4 **caractérisé en ce que** les fréquences micro-ondes MO sont comprises entre 300 MHz et 30GHz, préférentiellement à 915 MHz qui représente une fréquence autorisée avec une tolérance de 1.4% ou à 2.45 GHz qui représente une fréquence autorisée avec une tolérance de 2%.

6. Procédé selon la revendication 4 **caractérisé en ce que** les hautes fréquences HF sont comprises entre 3 MHz et 300MHz, préférentiellement à 13.56 MHz qui représente une fréquence autorisée avec une tolérance de 0.05% ou à 27.12 MHz qui représente une fréquence autorisée avec une tolérance de 0.6%.

7. Procédé selon l'une quelconque des revendications 1 à 6 **caractérisé en ce que** l'alcool polyhydrique de départ est choisi parmi :
le glycérol, le sorbitol, le sucrose, le mannitol, le xylitol, le néopentylglycol, le pentaérythritol, le saccharose, la galactose, le glucose, le maltose, le maltotriose, le fructose, le maltitol, le lactitol, le lactose, le ribose, le mellibiose, le cellobiose, le gentiobiose, l'altrose, le gulose, les polyalkylèneglycols, les polyglycérols, les polyphénols, les alkylpolyglucosides, les polyglucosides, le glycol, l'éthane 1,2 diol, le 1,4 butanediol, 1,6 hexanediol, les aminoalcools (par exemple la diéthanolamine DEA la triéthanolamine TEA, 3-amino 1,2 propanediol).

8. Procédé selon la revendication 7 **caractérisé en ce que** l'alcool polyhydrique est le glycérol.

9. Procédé selon l'une quelconque des revendications 1 à 8 **caractérisé en ce que** le temps de réaction dépend du polyglycérol que l'on souhaite obtenir et du degré de polymérisation attendu.

10. Procédé selon l'une quelconque des revendications 1 à 9 **caractérisé en ce qu'**il est mis en oeuvre avec ou sans catalyseurs.

11. Procédé selon l'une quelconque des revendications 1 à 10 **caractérisé en ce qu'**on ajoute au réactif ou au mélange réactionnel des catalyseurs hétérogènes ou homogènes, et de préférence des catalyseurs basiques.

12. Procédé selon la revendication 10 **caractérisé en ce qu'**on ajoute au réactif ou au mélange réactionnel des catalyseurs répondant aux fréquences radio ou aux fréquences micro-ondes.

13. Procédé selon l'une quelconque des revendications 1 à 12 **caractérisé en ce que** le réactif ou le mélange réactionnel, et éventuellement le(s) catalyseur(s), est placé dans un réacteur de type batch ou discontinu adapté pour recevoir des fréquences micro-ondes ou fréquences radio.

14. Procédé selon l'une quelconque des revendications 1 à 13 **caractérisé en ce que** le réactif ou le mélange réactionnel, et éventuellement le(s) catalyseur(s), est placé dans un réacteur adapté pour faire des réactions en continu.

15. Procédé selon l'une quelconque des revendications 1 à 14 **caractérisé en ce qu'**il comporte une polycondensation sous atmosphère normale ou riche en oxygène ou de préférence inerte ; sous pression atmosphérique ou sous pression réduite, de préférence entre 50 et 10 mm de mercure ; en renouvelant régulièrement l'atmosphère, sous agitation permanente.

16. Procédé selon la revendication 14 **caractérisé en ce qu'**il comporte une étape d'arrêt de la réaction de polycondensation en laissant refroidir ou en refroidissant le réactif ou le mélange réactionnel.

17. Procédé selon l'une quelconque des revendications 1 à 15 **caractérisé en ce que** les réactifs sont choisis parmi les alcools polyhydriques, d'origine naturelle ou synthétiques.

18. Procédé selon l'une quelconque des revendications 1 à 17 **caractérisé en ce qu'**on utilise un réactif isolé ou un mélange réactionnel comportant deux ou plusieurs composants, ces mélanges réactionnels comportant des proportions équivalentes de chaque composant ou certains composants peuvent être majoritaires.

19. Procédé selon l'une quelconque des revendications 1 à 18 **caractérisé en ce que** les réactifs comportant ou générant des fonctions hydroxyles sont choisis parmi les alcools, les aminoalcools, les époxydes.

20. Procédé selon l'une quelconque des revendications 1 à 19 **caractérisé en ce que**, parmi les catalyseurs ou adjuvants, on utilise les catalyseurs acides usuels (acide paratoluènesulfonique, acide sulfurique, acide phosphorique, acide perchlorique), les catalyseurs basiques usuels (soude, potasse, alcoolate de métaux alcalins et de métaux alcalino-terreux, acétate de sodium, triéthylamines, dérivés de pyridine), les résines acides et/ou basiques de type Amberlite™, Amberlyst™, Purolite™, Dowex™, Lewatit™, les zéolithes et les enzymes, les noirs de carbone et les fibres de carbone activées.

21. Procédé selon l'une quelconque des revendications 1 à 20 **caractérisé en ce que** les produits sont partiellement transestérifiés avec des esters d'acides gras saturés et/ou insaturés, des huiles végétales ou avec des huiles végétales polymérisées ou oxypolymérisées ou ils peuvent être partiellement estérifiés avec des acides gras saturés ou insaturés en cours de synthèse ou en fin de synthèse.

## Patentansprüche

1. Polykondensation-Verfahren, **dadurch gekennzeichnet, dass** es aufweist die dielektrische Erwärmung von mehrwertigen Alkoholen zum Erhalten von Polymeren von mehrwertigen Alkoholen und ist **dadurch gekennzeichnet, dass**:
- die Reaktionszeit, welche abhängig ist von der verwendeten Vorrichtung, der erreichten Temperatur und des Produkts, welches man zu erhalten wünscht (anders gesagt des Polymerisationsgrades, welchen man zu erreichen wünscht), zwischen 1 Stunde und 10 Stunden liegt,
- die in der dielektrischen Erwärmung verwendeten Frequenzen der elektromagnetischen Wellen von 30 Ghz bis 3 Mhz variieren,
- die Temperatur, der das Reagenz oder die Reaktionsmischung und eventuell der/die Katalysator/en ausgesetzt ist, zwischen 60 und 300 °C, vorzugsweise zwischen 160 und 260 °C liegt und
- die Anstiegszeit der Temperatur ausgewählt ist zwischen 3 und 180 Minuten.

2. Verfahren gemäß Anspruch 1, **dadurch gekennzeichnet, dass** es kein Lösemittel verwendet.

3. Verfahren gemäß Anspruch 1, **dadurch gekennzeichnet, dass** die dielektrische Heizung vorzugsweise realisiert wird in einer Atmosphäre ohne Sauerstoff.

4. Verfahren gemäß Anspruch 1, **dadurch gekennzeichnet, dass** die dielektrische Heizung bewerkstelligt wird durch die Verwendung von Frequenzen ausgewählt aus den Mikrowellen-Frequenzen oder Hochfrequenzen.

5. Verfahren gemäß Anspruch 4, **dadurch gekennzeichnet, dass** die Mikrowellen-Frequenzen MO zwischen 300 MHz und 30 GHz liegen, vorzugweise bei 915 MHz, die eine Frequenz darstellt, welche zulässig ist mit einer Toleranz von 1,4%, oder bei 2,45 GHz, die eine Frequenz darstellt, welche zulässig ist mit einer Toleranz von 2%.

6. Verfahren gemäß Anspruch 4, **dadurch gekennzeichnet, dass** die hohen Frequenzen HF zwischen 3 MHz und 300 MHz liegen, vorzugsweise bei 13,56 MHz, die eine Frequenz darstellt, welche zulässig ist mit einer Toleranz von 0,05%, oder bei 27,12 GHz, die eine Frequenz darstellt, welche zulässig ist mit einer Toleranz von 0,6%.

7. Verfahren gemäß irgendeinem der Ansprüche 1 bis 6, **dadurch gekennzeichnet, dass** der mehrwertige Alkohol vom Anfang ausgewählt ist aus:
Glycerol, Sorbitol, Sucrose, Mannitol, Xylitol, Neopentylglykol, Pentaerythritol, Saccharose, Galaktose, Glukose, Maltose, Maltotriose, Fruktose, Maltitol, Laktitol, Laktose, Ribose, Mellibiose, Cellobiose, Gentiobiose, Altrose, Gulose, Polyalkylenglykolen, Polyglycerolen, Polyphenolen, Alkylpolyglucosiden, Polyglucosiden, Glykol, Ethan-1,2-diol, 1,4-Butandiol, 1,6-Hexandiol, Aminoalkoholen (zum Beispiel Diethanolamin DEA, Triethanolamin TEA, 3-Amino-1,2-propandiol).

8. Verfahren gemäß Anspruch 7, **dadurch gekennzeichnet, dass** der mehrwertige Alkohol Glycerol ist.

9. Verfahren gemäß irgendeinem der Ansprüche 1 bis 8, **dadurch gekennzeichnet, dass** die Reaktionszeit vom Polyglycerol, welches man zu erhalten wünscht, und vom erwarteten Polymerisationsgrad abhängt.

10. Verfahren gemäß irgendeinem der Ansprüche 1 bis 9, **dadurch gekennzeichnet, dass** es durchgeführt wird mit oder ohne Katalysatoren.

11. Verfahren gemäß irgendeinem der Ansprüche 1 bis 10, **dadurch gekennzeichnet, dass** man dem Reagenz oder der Reaktionsmischung heterogene oder homogene Katalysatoren und vorzugsweise basische Katalysatoren zufügt.

12. Verfahren gemäß Anspruch 10, **dadurch gekennzeichnet, dass** man dem Reagenz oder der Reaktionsmischung Katalysatoren zufügt, welche auf Radio-Frequenzen oder auf Mikrowellen-Frequenzen ansprechen.

13. Verfahren gemäß irgendeinem der Ansprüche 1 bis 12, **dadurch gekennzeichnet, dass** das Reagenz oder die Reaktionsmischung und eventuell der/die Katalysator/en platziert werden in einem Batch- oder Chargen-Reaktor, welcher angepasst ist an das Empfangen von Mikrowellen-Frequenzen oder Radio-Frequenzen.

14. Verfahren gemäß irgendeinem der Ansprüche 1 bis 13, **dadurch gekennzeichnet, dass** das Reagenz oder die Reaktionsmischung und eventuell der/die Katalysator/en platziert ist in einem Reaktor, welcher angepasst ist an kontinuierliches Durchführen von Reaktionen.

15. Verfahren gemäß irgendeinem der Ansprüche 1 bis 14, **dadurch gekennzeichnet, dass** es eine Polykondensation aufweist unter normaler oder Sauerstoff-reicher oder vorzugsweise inerter Atmosphäre, unter atmosphärischem Druck oder reduziertem Druck, vorzugsweise zwischen 50 und 10 mm Quecksilber, unter regelmäßiger Erneuerung der Atmosphäre, unter ständiger Bewegung.

16. Verfahren gemäß Anspruch 14, **dadurch gekennzeichnet, dass** es aufweist einen Schritt des Reaktionsstopps der Polykondensation durch Erkalten-lassen oder Abkühlen des Reagenzes oder der Reaktionsmischung.

17. Verfahren gemäß irgendeinem der Ansprüche 1 bis 15, **dadurch gekennzeichnet, dass** die Reagenzien ausgewählt sind aus den mehrwertigen Alkoholen natürlichen oder synthetischen Ursprungs.

18. Verfahren gemäß irgendeinem der Ansprüche 1 bis 17, **dadurch gekennzeichnet, dass** man ein einzelnes Reagenz oder eine Reaktionsmischung, welche zwei oder mehrere Bestandteile aufweist, verwendet, wobei die Reaktionsmischungen aufweisen Proportionen, die von jedem Bestandteil gleich sind, oder bestimmte Bestandteile können mehrheitlich sein.

19. Verfahren gemäß irgendeinem der Ansprüche 1 bis 18, **dadurch gekennzeichnet, dass** die Reagenzien, welche Hydroxyl-Funktionen aufweisen oder generieren, ausgewählt sind aus Alkoholen, Aminoalkoholen, Epoxiden.

20. Verfahren gemäß irgendeinem der Ansprüche 1 bis 19, **dadurch gekennzeichnet, dass** man unter den Katalysatoren oder Zusatzstoffen verwendet die üblichen, sauren Katalysatoren (Paratoluolsulfonsäure, Schwefelsäure, Phosphorsäure, Perchlorsäure), die üblichen, basischen Katalysatoren (Natronlauge, Kalilauge, Alkali- und Erdalkalimetallalkoholate, Natriumacetat, Triethylamine, Pyridinderivate), die sauren und/oder basischen Reste des Typs Amberlite™, Amberlyst™, Purolite™, Dowex™, Lewatit™, Zeolithe und Enzyme, Ruße und aktivierte Kohlefasern.

21. Verfahren gemäß irgendeinem der Ansprüche 1 bis 20, **dadurch gekennzeichnet, dass** die Produkte partiell transesterifiziert sind mit gesättigten und/oder ungesättigten Fettsäureestern, Pflanzenölen oder mit polymerisierten oder oxypolymerisierten Pflanzenölen oder sie können partiell esterifiziert sein mit gesättigten oder ungesättigten Fettsäuren im Laufe der Synthese oder am Ende der Synthese.

## Claims

1. Polycondensation method, **characterised in that** it comprises the dielectric heating of polyhydric alcohols to obtain polymers of polyhydric alcohols; and is **characterised in that**:
- the reaction time, which is a function of the device used, of the temperature achieved and of the product which is to be obtained (in other words of the degree of polymerisation which is to be achieved), is between 1 hour and 10 hours;
- the frequencies of the electromagnetic waves used in the dielectric heating vary from 30 GHz to 3 MHz;
- the temperature to which the reagent or the reaction mixture, and optionally the catalyst(s), is subjected is between 60 and 300°C, preferably between 160 and 260°C; and
- the time in which temperature is increased is chosen between 3 and 180 minutes.

2. Method according to claim 1, **characterised in that** it does not employ a solvent.

3. Method according to claim 1, **characterised in that** the dielectric heating is carried out preferably in an oxygen-free atmosphere.

4. Method according to claim 1, **characterised in that** the heating is carried out by using frequencies chosen from the microwave or high-frequency frequencies.

5. Method according to claim 4, **characterised in that** the microwave frequencies MO are between 300 MHz and 30 GHz, preferably 915 MHz, which represents a frequency permitted with a tolerance of 1.4%, or 2.45 GHz, which represents a frequency permitted with a tolerance of 2%.

6. Method according to claim 4, **characterised in that** the high frequencies HF are between 3 MHz and 300 MHz, preferably 13.56 MHz, which represents a frequency permitted with a tolerance of 0.05%, or 27.12 MHz, which represents a frequency permitted with a tolerance of 0.6%.

7. Method according to any one of claims 1 to 6, **characterised in that** the starting polyhydric alcohol is chosen from:
glycerol, sorbitol, sucrose, mannitol, xylitol, neopentyl glycol, pentaerythritol, saccharose, galactose, glucose, maltose, maltotriose, fructose, maltitol, lactitol, lactose, ribose, mellibiose, cellobiose, gentiobiose, altrose, gulose, polyalkylene glycols, polyglycerols, polyphenols, alkyl polyglucosides, polyglucosides, glycol, ethane-1,2-diol, 1,4-butanediol, 1,6-hexanediol, amino alcohols (for example diethanolamine DEA, triethanolamine TEA, 3-amino-1,2-propanediol).

8. Method according to claim 7, **characterised in that** the polyhydric alcohol is glycerol.

9. Method according to any one of claims 1 to 8, **characterised in that** the reaction time depends on the polyglycerol which is to be obtained and on the expected degree of polymerisation.

10. Method according to any one of claims 1 to 9, **characterised in that** it is carried out with or without catalysts.

11. Method according to any one of claims 1 to 10, **characterised in that** heterogeneous or homogeneous catalysts, and preferably basic catalysts, are added to the reagent or to the reaction mixture.

12. Method according to claim 10, **characterised in that** catalysts are added to the reagent or to the reaction mixture corresponding to the radio frequencies or to the microwave frequencies.

13. Method according to any one of claims 1 to 12, **characterised in that** the reagent or the reaction mixture, and optionally the catalyst(s), is placed in a batch- or discontinuous-type reactor adapted to receive microwave frequencies or radio frequencies.

14. Method according to any one of claims 1 to 13, **characterised in that** the reagent or the reaction mixture, and optionally the catalyst(s), is placed in a reactor adapted to carry out continuous reactions.

15. Method according to any one of claims 1 to 14, **characterised in that** it comprises polycondensation under a normal or oxygen-rich or preferably inert atmosphere; under atmospheric pressure or under reduced pressure, preferably between 50 and 10 mm of mercury; the atmosphere being renewed regularly, with permanent stirring.

16. Method according to claim 14, **characterised in that** it comprises a step of stopping the polycondensation reaction by allowing the reagent or the reaction mixture to cool or by cooling the reagent or the reaction mixture.

17. Method according to any one of claims 1 to 15, **characterised in that** the reagents are chosen from polyhydric alcohols of natural origin or synthetic polyhydric alcohols.

18. Method according to any one of claims 1 to 17, **characterised in that** there is used a single reagent or a reaction mixture comprising two or more components, these reaction mixtures comprising equivalent proportions of each component or certain components may be in the majority.

19. Method according to any one of claims 1 to 18, **characterised in that** the reagents comprising or generating hydroxyl functions are chosen from alcohols, amino alcohols, epoxides.

20. Method according to any one of claims 1 to 19, **characterised in that**, among the catalysts or adjuvants, there are used conventional acidic catalysts (paratoluenesulfonic acid, sulfuric acid, phosphoric acid, perchloric acid), conventional basic catalysts (sodium hydroxide, potassium hydroxide, alkali metal and alkaline earth metal alcoholate, sodium acetate, triethylamines, pyridine derivatives), acidic and/or basic resins of the type Amberlite™, Amberlyst™, Purolite™, Dowex™, Lewatit™, zeolites and enzymes, carbon blacks and activated carbon fibres.

21. Method according to any one of claims 1 to 20, **characterised in that** the products are partially transesterified with saturated and/or unsaturated fatty acid esters, vegetable oils or with polymerised or oxypolymerised vegetable oils, or they can be partially esterified with saturated or unsaturated fatty acids during synthesis or at the end of synthesis.
